# EUROPEAN PATENT APPLICATION

(11) **EP 1 618 892 A1**
(43) Date of publication of application: **25.01.2006**
(21) Application number: 04729501.9
(22) Date of filing: 26.04.2004
(51) Int. Cl.: A61K 45/00, A61K 35/74, A61K 47/32, A61K 47/34, A61K 47/38, A61K 47/48, A61P 19/02, A61P 29/00, A61P 37/02, A61P 37/08, A61P 35/00, A61P 37/04, A61P 43/00

(54) **INSTRUMENT FOR INDUCING CYTOKINE AND METHOD OF INDUCING CYTOKINE**

(30) Priority: 28.04.2003 JP 2003124340; 28.04.2003 JP 2003124345
(71) Applicant: SEKISUI CHEMICAL CO., LTD., Osaka-shi, Osaka 530-8565 (JP)
(72) Inventor: SHINMURA, Kazuo, c/o SEKISUI CHEMICAL CO., LTD., Mishima-gun, Osaka 6188589 (JP); KITAHARA, Shinichiro, c/o SEKISUI CHEMICAL CO. LTD, Mishima-gun, Osaka 6188589 (JP); ABE, Yoshiko, c/o SEKISUI CHEMICAL CO., LTD., Mishima-gun, Osaka 6188589 (JP); KURIYAMA, Kiyoshi, c/o SEKISUI CHEMICAL CO., LTD., Mishima-gun, Osaka 6188589 (JP)
(74) Representative: Merkle, Gebhard
(86) International application number: PCT/JP2004/005986
(87) International publication number: WO 2004/096275

(57) **Abstract**

There are provided a cytokine-inducing instrument and a cytokine-inducing method, which can more effectively induce cytokine as compared with the previous cytokine-inducing method. A cytokine-inducing instrument comprising a cytokine-inducing agent, and a carrier of a water-insoluble porous material, and cytokine-inducing method for inducing cytokine from a cell producing cytokine, using the cytokine-inducing instrument.

## Description

### TECHNICAL FIELD

The present invention relates to a cytokine-inducing instrument and a cytokine-inducing method which are used in cytokine induction therapy and can effectively induce cytokine.

### BACKGROUND ART

Cytokine is a general term for a diversity of factors of cell signal transductions. Examples of cytokine include interferon α, interferon β, interferon γ (IFN-γ), interleukin 1 to interleukin 27, tumor necrosis factor-α (TNF-α), tumor necrosis factor-β, transforming growth factor-α, transforming growth factor-β (TGF-β) and various cell growth factors (Clinical Immunology, vol.27, Suppl.16, "All of Cytokines", Kagakuhyoronsha Publishing Co., Clinical Immunology, vol.36, 39-44, 2001, Clinical Immunology, vol.39, 189-2000, 2003).

It is known that cytokine has a variety of activities in vivo, and is involved in various diseases. Cytokine induction therapy has previously been performed in which activity of such the cytokine is induced in vivo, and a disease is treated. In cytokine induction therapy, a cytokine-inducing agent is administered to a patient, and induction of cytokine is caused in vivo. As a cytokine-inducing substance used in such the cytokine induction therapy, a variety of substances are known. For example, microorganism-derived OK-432, Mycobacteria bovis bacillus Calmette-Guerin(BCG), Bestatin, SSM(Supecific Substance Maruyama), and Romurtide are known and, as a cytokine-inducing substance derived from basidiomycetes, Krestin, Lentinan, and Sizofiran are known.

For example, it is known that OK-432 and BCG induce cytokine such as interleukin 1 and interferon γ from blood (Acta Sch. Med. Univ. Gifu 43:166-177, 1995, Molecular Medicine vol.36, extra issue 220-229, 1999).

In the aforementioned cytokine induction therapy, cytokine can be induced in vivo, but there is a problem that it is difficult to induce a sufficient amount of cytokine, and it is difficult to exert strong efficacy. In addition, there is also a problem that, in order to effectively induce cytokine, a dose of a cytokine-inducing agent is increased, side effect becomes serious, and therapy can not be performed effectively.

Japanese Patent Application Laid Open (JP-A) No.61-277628 describes a leukocyte stimulating material for treating cancer in which OK-432 which is one kind of hemolytic streptococci is covalently bound with an insoluble carrier. However, this induces a tumor cytotoxic cell and, in this prior art reference, a cytokine-inducing instrument and a cytokine-inducing method are not described at all.

On the other hand, a method of contacting blood and an insoluble material using an extracorporeal circulation system outside a body has been also studied. The previous extracorporeal circulation system is for the purpose of removing a harmful substance of blood.

In addition, a blood bag used in blood transfusion is used only for the purpose of storing and preserving blood or blood components. That is, under the current circumstances, a cytokine-inducing instrument and a cytokine-inducing method which positively induce production of cytokine are not known at all.

### DISCLOSURE OF THE INVENTION

In view of the aforementioned circumstances, an object of the present invention is to provide a novel cytokine-inducing instrument and a cytokine-inducing method, which can effectively induce cytokine as compared with the previous cytokine induction therapy.

The present invention is a cytokine-inducing instrument containing a cytokine-inducing agent, and a carrier consisting of a water-insoluble porous material, and a cytokine-inducing method. It is preferable that the carrier is an induction enhancing material which enhances cytokine-inducing action of a cytokine-inducing agent. It is further preferable that the induction enhancing material which enhances cytokine-inducing action enhances induction of IFN-γ.

The present inventors found out that a cytokine-inducing instrument containing a carrier consisting of a water-insoluble porous material together with a cytokine-inducing agent, or a cytokine-inducing instrument consisting of an insoluble carrier water on which a cytokine-inducing agent is immobilized exhibits a remarkably high cytokine-inducing amount, which resulted in completion of the present invention.

The present invention will be described in detailed below.

The cytokine-inducing instrument of the present invention contains a cytokine-inducing agent, and a carrier consisting of a water-insoluble porous material.

The carrier is not particularly limited as far as it consists of a water-insoluble porous material, but for example is constructed of a metal, an organic entity or an inorganic entity, preferably of an organic material, more preferably a polymer material.

Examples of the metal include gold or a gold alloy, silver or a silver alloy, titanium or a titanium alloy, and stainless.

Examples of the inorganic entity include active carbon, a glass or a glass derivative, a silica-based composition, alumina, and hydroxyapatite.

Examples of the organic material or polymer material include cellulose-based, agarose-based, dextran-based, polystyrene-based, acrylester-based, polyethylene terephthalate-based, nylon-based, polyvinyl alcohol-based, polysulfone-base, polyamide-based, polyacrylonitrile-based, polyethylene-based, polyurethane-based, polypropylene-based, and polyester-based materials.

Examples of the polystyrene-based material include a divinylbenzene-styrene copolymer, and examples of the acrylester-based material include polymethyl methacrylate, and polyhydroxyethyl methacrylate.

As the carrier, inter alia, a carrier consisting of a polystyrene-based or acrylester-based polymer material is preferable.

The carrier is non-polar, and may be hydrophobic and, in this case, as a carrier, a polystyrene-based polymer material can be used. In addition, hydrophobicity may be imparted to a surface of these carriers by surface modification, surface coating or the like.

When immobilization of the cytokine-inducing agent on the carrier is performed by a physical adsorption method, the carrier consists of preferably a material which has not an ion exchangeable functional group or a hydrophilic functional group, and has a surface having strong hydrophobicity, more preferably a polymer material consisting of styrene which has not an ion exchangeable functional group or a hydrophilic functional group, particularly preferably a polymer material using a crosslinking agent such as divinylbenzene in styrene.

A form of the carrier is not particularly limited, but the known shape such as fiber-like, non-woven fabric-like, sponge-like, particulate, film-like, and hollow fiber-like shapes can be used.

As a size of the carrier, a preferable lower limit is 50µm, and a preferable upper limit is 2mm in the case of particulate shape, and a fiber diameter is preferably 10µm or smaller, more preferably 5µm or smaller in the case of fiber-like shape.

When the carrier is fiber-like, the carrier consists of preferably a non-woven fabric, and a fiber diameter is preferably 3µm or smaller.

Further, it is considered that it is preferable to use a carrier onto which leukocyte is not adsorbed in a leukocyte stimulating material for treating cancer in JP-A No.61-277628, while the carrier of the present invention consists of preferably a material onto which leukocyte is adsorbed, and examples of a material onto which leukocyte is adsorbed include polymer materials consisting of a polystyrene-based material, an acrylester-based material, a polyester-based material, a nylon-based material, a polyvinyl alcohol-based material, or a cellulose-based material such as cellulose acetate, and glass-based materials.

The carrier consisting of a porous polymer material is preferable.

As a form of a carrier, spherical or non-spherical various shapes may be used, and the shape is not particularly limited. Examples of the non-spherical shape include a pellet-like shape, a pillar shape, a cylinder-like shape, a ground-like shape, a fiber-like shape, a sponge-like shape, a hollow fiber-like shape, and a sheet-like shape. Alternatively, a material in which a porous material is imparted to an internal surface of a container, or the aforementioned shape by coating or the like, may be used.

A surface area of the porous polymer material is preferably 400m²/g or more, a pore volume is preferably 0. 5mL/g, and an average pore diameter is preferably such that a lower limit is 20Å, and an upper limit is 800Å.

The porous polymer material is roughly classified into a gel type having a standard gel structure, and a MR type having a macro reticular structure. Since a porous resin obtained by simple polymerization using styrene and a crosslinking agent such as divinylbenzene is approximately transparent, and exhibits a gel structure, it is called gel-type porous resin. On the other hand, according to a special polymerization method, a physically porous resin or the like can be prepared, and such the resin is called MR-type or macro porous-type porous resin. Further, by using a special organic solvent which is insoluble in water and can dissolve only a monomer upon suspension polymerization, a MR-type or macro porous-type particle having a large pore (macropore) until a deep inner part of a particle can be prepared.

Examples of a particulate porous polymer material include materials consisting of a strongly acidic cation exchange resin, a weakly acidic cation exchange resin, a strongly basic anion exchange resin, a weakly basic anion exchange resin, a synthetic adsorbing agent or a chelate resin. These porous particles can be obtained by the known method, and can be prepared as a spherical polymer by suspension-polymerizing a raw material monomer in water using a suitable suspension stabilizer such as gelatin. As the raw material monomer, a compound having an ethylenic double bond such as styrene, vinyltoluene, ethyl acrylate, methyl methacrylate, and acrylonitrile is used, to this is added a crosslinking agent which is a polyfunctional monomer such as divinylbenzene and ethylene dimethacrylate, and a polymerization initiator such as benzoyl peroxide and azobisisobutyronitrile is used in the presence of a pore-forming agent to obtain a crosslinked polymer. Examples of a commercially available particle among the aforementioned porous particle include Diaoin (registered trademark) manufactured by Mitsubishi Chemical Corporation, Amberlite manufactured by Rohm & Haas, and Dowex manufactured by Dow Chemical.

For example, a spherical resin having a crosslinked bond can be made by suspending a monomer of styrene and divinylbenzene (crosslinking agent) in water, and polymerizing it. When a suitable solvent which dissolves a monomer is added thereupon, phase separation is induced during a process of polymerization and, as a result, a polymer material having a porosity in such a range that can be measured by the following pore size distribution measuring method is prepared.

As the carrier, a porous polymer material having a macro reticular structure is more preferable. As a pore size distribution of the macro reticular structure, it is preferable that a lower limit is 2Å, and an upper limit is 2000Å, and a more preferable upper limit is 300Å. A pore size distribution can be defined by a generally used method and, for example, can be shown by a pore diameter occupied by 5 to 95% of a pore volume.

A pore size distribution can be measured by a general test method which has previously been used. Examples of such the test method include a mercury penetration method, a gas adsorbing method and a bubble point method. In the mercury penetration method, generally, a measuring apparatus called mercury penetration-type porosimeter is used. This principle is that a sample is placed into a container in which vacuum treatment can be performed, per-treatment of removing water and various gasses placed in a fine pore is performed, and a sample is covered with mercury. And, as a pressure is applied to entire mercury, a place to which mercury is escaped is lost, and mercury enters a fine pore formed on a surface of a sample. Since as a pressure is raised, mercury enters a smaller pore (that is, since a pressure and a size of a pore are inversely proportional), when an applied pressure and an amount of a change in mercury are investigated, a graph of a relationship (pore size distribution curve) between a pore size (abscissa) and a pore volume (ordinate) can be obtained.

In addition, in the gas adsorbing method, generally, using a gas such as nitrogen, helium and krypton, as in the aforementioned porosimeter, when a sample is degassed in a vacuum apparatus, and a constant pressure or a constant amount of a gas is added to a sample container, a gas molecule is adsorbed onto a surface of a sample. In the case of a mercury penetration method, mercury enters a large pore first, but in the gas adsorbing method, a gas is adsorbed in a small pore first. When a gas is added to a sample by dividing into a few tens times, saturation of adsorption is repeated each time, and the state where no adsorption is seen even when more gas is added is realized. And, a pore size distribution until a relatively small pore can be measured.

The bubble point method is generally a method of measuring a pore size distribution of a through hole, and is suitable in a filter or a non-woven fabric. As a sample wetted with flon is fixed, and a nitrogen gas is pressurized, a flon solution attached to a large pore is flown at a certain pressure, and this is flown as a bubble. And, abruptly, a gas is flown out upwardly from the pore. Further, when a more gas is struck at a sample, a flon solution is flown again as a bubble from a smaller pore, and a gas is flown out, at a higher pressures than that of first time. When a pressure of a gas and an amount of a flown gas thereupon are measured, the same pore size distribution measurement as that of a porosimeter can be performed.

A method of measuring a pore size distribution is not limited to the aforementioned method, but other method which can measure a distribution of a pore size may be used.

Examples of the cytokine-inducing agent include bacteria and components thereof such as BCG, BCG-CWS, PPD(Purified protein derivatives Tuberculin), Nocardia-CWS, OK-432, and Muramyldipeptide; polysaccarides such as PSK(Krestin), Lentinan, and Sizofiran; polymers such as polyI : C, and polyA:U; chemical substances such as Levamisole, DNCB, Azimexon, Tilorone, and Bestatin. The agent is not limited to the aforementioned physiologically active substances, but various substances such as bacterium cells, bacterial cell components, mycobacteria, hemolyticstreptococcus, actinomycete, peptides, nucleic acids, proteins, sugars, lipids, prostaglandins, arachidonic acid methabolites, and proteins such as Keyhole Limpet Hemocyanin (KLH) can be also used as a cytokine-inducing agent.

Among these cytokine-inducing agents, bacterium cells and/or components derived from the bacterium cells are preferable. Among them, mycobacteria and components derived from mycobacteria are more preferable and tubercle bacillus and components derived from tubercle bacillus are particularly preferable. BCG which is mycobacterium bovis attenuated strain, and components derived from it are also particularly preferable.

In addition, as the cytokine-inducing agent, hemolytic streptococcus and/or components derived from hemolytic streptococcus are also preferable.

In addition, the aforementioned cytokine-inducing agent which itself cannot sufficiently exert cytokine-inducing ability, by using by combining with the aforementioned carrier, can also exert cytokine-inducing activity. Therefore, as the cytokine-inducing agent in the present invention, in addition to cytokine-inducing substances which have previously been used, a variety of substances can be used.

In order to immobilize the cytokine-inducing agent on a surface of the carrier, the known methods such as physical adsorption, covalent bonding, and ion bonding can be used. In addition, when immobilization is performed by covalent bonding, if necessary, it is preferable to introduce a spacer having an arbitrary length into a bonding part between the cytokine-inducing agent and the carrier.

When the cytokine-inducing agent is bacterium cells, if necessary, before immobilization, various pre-treatment such as operation of washing or grinding bacterium cells, and operation of fractionating components may be performed. In addition, when the cytokine-inducing agent is a live bacterium, in order to further enhance safety, if necessary, at any time of before immobilization, at the same time with immobilization, and after immobilization, the bacterium may be converted into a dead bacterium by various methods such as heat treatment, chemical treatment, radiation treatment, and gas sterilizing treatment. Examples of the heat treatment include autoclave treatment, examples of the chemical treatment include glutaraldehyde treatment, formalin treatment and ethanol treatment, examples of the radiation treatment include γ-ray treatment, and examples of the gas sterilizing treatment include ethylene oxide gas treatment.

As the cytokine-inducing agent, an agent denatured with a protein denaturing agent is suitably used. Examples of the protein denaturing agent include various solvents such as glutaraldehyde, formalin, and ethanol, and surfactants. In addition, since heat treatment exerts protein denaturing action, heat treatment in an aqueous solution is included in the protein denaturing agent. Inter alia, a cytokine-inducing agent denatured with formalin is more preferable.

When the cytokine-inducing agent is a microorganism such as BCG, it can be bound to a functional group such as a carboxyl group, an amino group and/or an epoxy group of the carrier via an amino acid or a sugar component of a bacterium cell surface outer wall component. Thereupon, if necessary, a spacer of various chain length and structures may be introduced.

When the cytokine-inducing agent is a microorganism in which a bacterium cell outer layer is covered with a lipid such as BCG, if necessary, a lipid is washed and removed, and may be bound to a functional group of the carrier.

As a method of immobilizing the cytokine-inducing agent on the carrier, a physical adsorbing method is preferable. For example, when the carrier has a hydrophobic surface, and the cytokine-inducing agent is BCG, the agent can be immobilized by physical adsorbing action. In addition, also when the cytokine-inducing agent is a microorganism or a component thereof, and a surface thereof is charged, the agent can be immobilized on a carrier having a counter-charge on a surface by physical adsorbing action.

A ratio of the carrier to be used is not particularly limited, but when used as a particulate carrier, as expressed by a bulk volume amount of a carrier relative to a blood volume, a lower limit is 0.02%, and an upper limit is around 80%, and a preferable lower limit is 0.1%, and a preferable upper limit is around 50%.

A ratio of the cytokine-inducing agent to be used is not particularly limited, but when the agent is BCG, as a concentration to be added to blood, as expressed by a dry weight, preferably, a lower limit is 0.001mg/mL, and an upper limit is 10mg/mL. In addition, when the cytokine-inducing agent is OK-432, as a concentration to be added to blood, preferably, a lower limit is 0.0001KE/mL, and an upper limit is 10KE/mL.

A cytokine-inducing instrument having the cytokine-inducing instrument of the present invention accommodated in a container is one of the present invention.

In the cytokine-inducing instrument of the present invention, a cytokine-inducing instrument containing the carrier and a cytokine-inducing agent is contacted with a cell producing cytokine in a container, thereby, cytokine is effectively induced. In this case, it is preferable that a contacting temperature is in such a range that a lower limit is 15°C, and an upper limit is 42°C, thereby, inducing of cytokine can be more effectively caused.

The carrier and the cytokine-inducing agent may be mixed in advance, and may be contacted with a cell producing cytokine, or may be separately contacted with a cell producing cytokine.

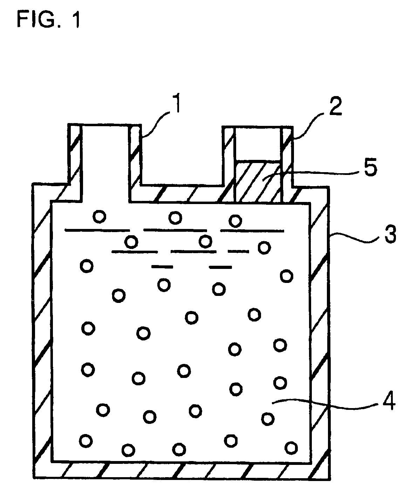

In addition, in the cytokine-inducing instrument of the present invention, a structure of a container is not particularly limited, but as schematically shown in Fig.1, a container 3 equipped with a part 1 for introducing blood, and an exit part 2 for guiding blood 4 contact with a cytokine-inducing instrument outside a container, is preferable.

As the container, a columnar container or a blood bag-like container is more preferable, and a blood bag-like container is further preferable.

In addition, in the blood bag-like container, since a time during which the carrier and/or cytokine-inducing agent is contacted with blood or blood components can be arbitrarily selected, necessary cytokine can be induced.

It is more preferable that the cytokine-inducing instrument of the present invention is provided with an overflow preventing mechanism so that a carrier and/or a cytokine-inducing agent is not mixed in blood when blood contact with the carrier and/or cytokine-inducing agent is guided to the outside of a container.

As schematically shown in Fig.1, a carrier and/or cytokine-inducing agent overflow preventing mechanism 5 may be such that the carrier is immobilized in an interior of a container 3, or for example, a separating membrane or a separating filter for preventing overflow may be provided.

As one embodiment of the cytokine-inducing instrument of the present invention, for example, a cytokine-inducing agent immobilized on a particulate, fibrous or non-woven fabric-like carrier is filled in a blood bag having an introducing part and an exit part, and blood or blood components are introduced therein. Thereafter, this is incubated at a prescribed temperature for a prescribed time, blood or blood components in which cytokine is induced is taken out through an exit part, and can be utilized in cytokine induction therapy by instillation.

A cytokine-inducing method which is carried out using the cytokine-inducing instrument of the present invention is also one of the present invention.

Cytokine which is induced by the cytokine-inducing instrument or the cytokine-inducing method of the present invention is not particularly limited, but inter alia, preferable examples include interferon γ(IFN-γ), interleukin 10 (IL-10), and tumor necrosis factor-α(TNF-α). For example, IFN-γ is cytokine having a very important role in various diseases such as allergic disease and cancer, and therapeutic effect for these diseases can be expected by inducing IFN-γ.

Examples of a cell which can produce cytokine in the cytokine-inducing instrument and the cytokine-inducing method of the present invention include blood, blood components, and various cells which can produce cytokine, are not limited to leukocyte and platelet separated from blood, but refer to cells taken from a tissue such as bone marrow cells, dendritic cells, epithelial cells, fibroblast, hepatocyte, osteoblast, blood stem cells, and embryonic stem cells, cultured cells, established cells, and various cells which can produce cytokine.

Blood in the cytokine-inducing instrument and the cytokine-inducing method of the present invention refers to blood, and an entity obtained by diluting blood with an appropriate diluent such as a physiological saline, a medium and a buffer. An anti-coagulant or an additive may be added to blood.

A blood component in the cytokine-inducing instrument and the cytokine-inducing method of the present invention is not limited to leukocyte and platelet separated from blood, but refers to an entity containing bone marrow cells, dendritic cells, blood stem cells, or blood cells-derived established cells. These blood components may be used alone, or separately taken out leukocyte may be used by mixing with blood. Alternatively, these cells may be diluted with an appropriate diluent such as a physiological saline, a medium or a buffer, which may be used in the present invention. Preferably, the blood is used.

In explanation in the text, in order to make explanation understandable, as a cell producing cytokine, an example using blood or a blood component is explained.

### BRIEF DESCRIPTION OF THE DRAWING

Fig.1 is a schematic cross-sectional view showing one example of the cytokine-inducing instrument of the present invention.

### BEST MODE FOR CARRYING OUT THE INVENTION

The present invention will be explained in more detail below by way of Examples, but the present invention is not limited to only these Examples.

IFN-γ in human plasma was measured with an ELISA kit manufactured by R & D Systems, an ELISA kit manufactured by ENDOGEN, or an ELISA kit manufactured by Japan Immunoresearch Laboratories Co., Ltd.

### (Example 1)

A carrier 1 (manufactured by Organo, trade name: Amberlite XAD4, porous polystyrene particle, MR type, pore size distribution 2 to 150Å, surface area 700m²/g or more, pore volume 0.5mL/g or more, average pore diameter about 120Å) was washed by decantation with purified water (manufactured by Otsuka Pharmaceutical Co., Ltd.) and, thereafter, washed by decantation with methanol (for HPLC, manufactured by Wako Pure Chemical Industries, Ltd.). Then, the carrier 1 was washed by decantation with a physiological saline for injection (manufactured by Otsuka Pharmaceutical Co., Ltd.), and the carrier 1 having a particle bulk volume of 50µL was filled into a sterilized tube (for Eppendorf tube 1.5mL, manufactured by Diatron).

Blood was taken from a healthy person, and heparin 15IU/mL-containing vein blood was obtained. BCG (manufactured by Japan BCG Manufacturing Co., Ltd.) was added to the blood to a concentration of 1mg/mL. BCG was prepared in a physiological saline. Thereupon, a volume of a physiological saline was adjusted to 1.25% relative to the blood.

About 1.45mL of blood with the BCG added was added to a tube filled with the carrier 1.

Then, the tube was subjected to inversion stirring of blood, this was attached to a rotary mixer (manufactured by TAIAEC),and incubated in a constant temperature bath at 37°C for 24 hours while inverted and kneaded at 6rpm. Blood after incubation was centrifuged at 4°C and 3500rpm (manufactured by Tomy Seiko Co., Ltd., minor amount high speed centrifuge MRX-150) for 15 minutes, thereafter, plasma was collected, and the plasma was freezing-stored at -70°C. Then, the stored plasma was thawed, and an amount of induced IFN-γ in plasma was measured with a Human IFN-γ ELISA kit (manufactured by R & D System or manufactured by ENDOGEN). The results are shown in Table 1.

In addition, immediately after collection of blood of a healthy person used and after culturing of only blood as described above, all values of IFN-γ in plasma were 10pg/mL or lower.

### (Comparative Example 1)

According to the same manner as that of Example 1 except that a carrier 1 was not used, and 1.5mL of blood with BCG added was used, an amount of induced IFN-γ was measured. The results are shown in Table 1.

### (Comparative Example 2)

According to the same manner as that of Example 1 except that a carrier 1 was not used, and 1.5mL of blood with no BCG added was used, an amount of induced IFN-γ was measured. The results are shown in Table 1.

### (Comparative Example 3)

According to the same manner as that of Example 1 except that BCG was not added to blood, an amount of induced IFN-γ was measured. The results are shown in Table 1.

### (Example 2)

According to the same manner as that of Example 1 except that a carrier 1 was changed to a carrier 2 (manufactured by Organo, trade name: Amberlite XAD16HP, polystyrene porous particle, MR type, pore size distribution 2 to 300Å, surface area 800m²/g or more, pore volume 0.58 to 0.65mL/g, average pore diameter about 150Å), an amount of induced IFN-γ was measured. The results are shown in Table 1.

### (Comparative Example 4)

According to the same manner as that of Example 2 except that BCG was not added to blood, an amount of induced IFN-γ was measured. The results are shown in Table 1.

### (Example 3)

According to the same manner as that of Example 1 except that a carrier 1 was changed to a carrier 3 (manufactured by Organo, trade name: Amberlite XAD2000, polystyrene porous particle, MR type, pore size distribution 2 to 80Å) surface area about 620m²/g, pore volume about 0.7mL/g, average pore diameter about 40 to 50Å), an amount of induced IFN-γ was measured. The results are shown in Table 1.

### (Comparative Example 5)

According to the same manner as that of Example 3 except that BCG was not added to blood, an amount of induced IFN-γ was measured. The results are shown in Table 1.

### (Example 4)

According to the same manner as that of Example 1 except that a carrier 1 was changed to a carrier 4 (manufactured by Organo, trade name: Amberlite XAD7HP, acrylic acid-based porous particle, MR type, pore size distribution 2 to 2000Å, surface area 400m²/g or more, pore volume 0.5mL/g or more, average pore diameter about 400 to 500Å), an amount of induced IFN-γ was measured. The results are shown in Table 1.

### (Comparative Example 6)

According to the same manner as that of Example 4 except that BCG was not added to blood, an amount of induced IFN-γ was measured. The results are shown in Table 1.

### (Comparative Example 7)

According to the same manner as that of Example 1 except that, as a carrier, trade name Polybeads Polystyrene Microsphere (particle diameter 500 to 600µm, non-porous) manufactured by Polysciences was used, an amount of induced IFN-γ was measured. The results are shown in Table 1.

### (Comparative Example 8)

According to the same manner as that of Example 1 except that, as a carrier, a spherical polystyrene particle (styrene-divinylbenzene copolymer, particle diameter 250 to 750µm, non-porous) manufactured by Moritex Corporation was used, an amount of induced IFN-γ was measured. The results are shown in Table 1.

### (Comparative Example 9)

According to the same manner as that of Example 1 except that, as a carrier, a spherical polymethyl methacrylate particle (particle diameter 750 to 1000µm, non-porous) manufactured by Moritex Corporation was used, an amount of induced IFN-γ was measured. The results are shown in Table 1.

### (Comparative Example 10)

According to the same manner as that of Comparative Example 3 except that, as a carrier, trade name Polybeads Polystyrene Microsphere (particle diameter 500 to 600µm, non-porous) manufactured by Polysciences was used, an amount of induced IFN-γ was measured. The results are shown in Table 1.

### (Comparative Example 11)

According to the same manner as that of Comparative Example 3 except that, as a carrier, a spherical polystyrene particle (styrene-divinylbenzene copolymer, particle diameter 250 to 750µm, non-porous) manufactured by Moritex Corporation was used, an amount of induced IFN-γ was measured. The results are shown in Table 1.

### (Comparative Example 12)

According to the same manner as that of Comparative Example 3 except that, as a carrier, a spherical polymethyl methacrylate particle (particle diameter 750 to 1000µm, non-porous) manufactured by Moritex Corporation was used, an amount of induced IFN-γ was measured. The results are shown in Table 1.

**Table 1**

| | | IFN-γ Concentration (pg/mL) |
|---|---|---|
| Example | 1 | 231 |
| | 2 | 181 |
| | 3 | 186 |
| | 4 | 142 |
| Comparative Example | 1 | 86 |
| | 2 | <10 |
| | 3 | <10 |
| | 4 | <10 |
| | 5 | <10 |
| | 6 | <10 |
| | 7 | 78 |
| | 8 | 72 |
| | 9 | 77 |
| | 10 | <10 |
| | 11 | <10 |
| | 12 | <10 |

### (Example 5)

In place of BCG, picibanil (manufactured by Chugai Pharmaceutical Co., ltd., OK-432) was added to each blood to a concentration of 0.1KE/mL. OK-432 was prepared in a physiological saline for injection (manufactured by Otsuka Pharmaceutical Co., Ltd.), and was prepared so that a physiological saline was 1% relative to blood. Blood with OK-432 added was added to the tube filled with a carrier 1 having a bulk volume of 20µL to a scale of a tube of 1.5mL. That is, blood was added at about 1.48mL.

Under other conditions as Example 1, an amount of induced IFN-γ was measured. The results are shown in Table 2.

In addition, immediately after collection of blood of a healthy person used and after culturing of only blood as described above, all values of IFN-γ in blood were 10pg/mL or lower.

### (Comparative Example 13)

According to the same manner as that of Example 5 except that a carrier 1 was not used, and 1.5mL of blood with OK-432 added was used, an amount of induced IFN-γ was measured. The results are shown in Table 2.

### (Comparative Example 14)

According to the same manner as that of Example 5 except that a carrier 1 was not used, and 1.5mL of blood with no OK-432 added was used, an amount of induced IFN-γ was measured. The results are shown in Table 2.

### (Comparative Example 15)

According to the same manner as that of Example 5 except that OK-432 was not added to blood, an amount of induced IFN-g was measured. The results are shown in Table 2.

### (Example 6)

According to the same manner as that of Example 5 except that a carrier 1 was changed to a carrier 2 (manufactured by Organo, trade name: Amberlite XAD16HP, polystyrene porous particle, MR type, pore size distribution 2 to 300Å, surface area 800m²/g or more, pore volume 0.58 to 0.65mL/g, average pore diameter about 150Å), an amount of induced IFN-g was measured. The results are shown in Table 2.

### (Comparative Example 16)

According to the same manner as that of Example 6 except that OK-432 was not added to blood, an amount of induced IFN-γ was measured. The results are shown in Table 2.

### (Example 7)

According to the same manner as that of Example 5 except that a carrier 1 was changed to a carrier 3 (manufactured by Organo, trade name: Amberlite XAD2000, polystyrene porous particle, MR type, pore size distribution 2 to 80Å, surface area about 620m²/g, pore volume about 0.7mL/g, average pore diameter about 40 to 50Å), an amount of induced IFN-γ was measured. The results are shown in Table 2.

### (Comparative Example 17)

According to the same manner as that of Example 7 except that OK-432 was not added to blood, an amount of induced IFN-γ was measured. The results are shown in Table 2.

### (Example 8)

According to the same manner as that of Example 5 except that a carrier 1 was changed to a carrier 4 (manufactured by Organo, trade name: Amberlite XAD7HP, acrylic acid-based porous particle, MR type, pore size distribution 2 to 2000Å, surface area 400m²/g or more, pore volume 0. 5mL/g or more, average pore diameter about 400 to 500Å), an amount of induced IFN-γ was measured. The results are shown in Table 2.

### (Comparative Example 18)

According to the same manner as that of Example 8 except that OK-432 was not added to blood, an amount of induced IFN-γ was measured. The results are shown in Table 2.

### (Comparative Example 19)

According to the same manner as that of Example 5 except that, as a carrier, trade name Polybeads Polystyrene Microsphere (particle diameter 500 to 600µm, non-porous) manufactured by Polysciences was used, an amount of induced IFN-γ was measured. The results are shown in Table 2.

### (Comparative Example 20)

According to the same manner as that of Example 5 except that, as a carrier, a spherical polystyrene particle (styrene-divinylbenzene copolymer, particle diameter 250 to 750µm, non-porous) manufactured by Moritex Corporation was used, an amount of induced IFN-γ was measured. The results are shown in Table 2.

### (Comparative Example 21)

According to the same manner as that of Example 5 except that, as a carrier, a spherical polymethyl methacrylate particle (particle diameter 750 to 1000µm, non-porous) manufactured by Moritex Corporation was used, an amount of induced IFN-γ was measured. The results are shown in Table 2.

### (Comparative Example 22)

According to the same manner as that of Comparative Example 15 except that, as a carrier, trade name Polybeads Polystyrene Microsphere (particle diameter 500 to 600µm, non-porous) manufactured by Polysciences was used, an amount of induced IFN-γ was measured. The results are shown in Table 2.

### (Comparative Example 23)

According to the same manner as that of Comparative Example 15 except that, as a carrier, a spherical polystyrene particle (styrene-divinylbenzene copolymer, particle diameter 250 to 750µm, non-porous) manufactured by Moritex Corporation was used, an amount of induced IFN-γ was measured. The results are shown in Table 2.

### (Comparative Example 24)

According to the same manner as that of Comparative Example 15 except that, as a carrier, a spherical polymethyl methacrylate particle (particle diameter 750 to 1000µm, non-porous) manufactured by Moritex Corporation was used, an amount of induced IFN-γ was measured. The results are shown in Table 2.

**Table 2**

| | | IFN-γ Concentration (pg/mL) |
|---|---|---|
| Example | 5 | 255 |
| | 6 | 236 |
| | 7 | 211 |
| | 8 | 192 |
| Comparative Example | 13 | 107 |
| | 14 | <10 |
| | 15 | <10 |
| | 16 | <10 |
| | 17 | <10 |
| | 18 | <10 |
| | 19 | 89 |
| | 20 | 86 |
| | 21 | 84 |
| | 22 | <10 |
| | 23 | <10 |
| | 24 | <10 |

### (Example 9)

A bulk volume of 1mL of a carrier 1 (manufactured by Organo, Amberlite XAD-4) and 1mL of BCG (10mg/mL) were kneaded in a physiological saline at 37°C for 20 hours, and BCG was physically adsorbed onto a particle surface of the carrier 1. After kneading, the carrier 1 was sufficiently washed with a physiological saline, and was suspended in a physiological saline again. The thus obtained carrier 1 having a bulk volume of 100µL was filled into a sterilized tube. Thereafter, according to the same manner as that of Example 1 except that 1. 4mL of blood was added to this tube, an amount of induced IFN-γ was measured with blood of a healthy person. The results are shown in Table 3.

In addition, immediately after collection of blood of a healthy person used and after culturing of only blood as described above, all values of IFN-γ in plasma were 10pg/mL or lower.

### (Example 10)

According to the same manner as that of Example 9 except that a carrier 1 was changed to a carrier 2 (manufactured by Organo, Amberlite XAD16HP), an amount of induced IFN-γ was measured with blood of a healthy person. The results are shown in Table 3.

### (Example 11)

According to the same manner as that of Example 9 except that a carrier 1 was changed to a carrier 3 (manufactured by Organo, Amberlite XAD2000), an amount of induced IFN-γ was measured with blood of a healthy person. The results are shown in Table 3.

### (Example 12)

According to the same manner as that of Example 9 except that a carrier 1 was changed to a carrier 4 (manufactured by Organo, Amberlite XAD7HP), an amount of induced IFN-γ was measured with blood of a healthy person. The results are shown in Table 3.

### (Comparative Example 25)

According to the same manner as that of Example 9 except that a carrier was not used, and 1.5mL of blood with BCG added was used, an amount of induced IFN-γ was measured. The results are shown in Table 3.

### (Comparative Example 26)

According to the same manner as that of Example 9 except that a carrier with no BCG physically adsorbed thereon was used, an amount of induced IFN-γ was measured. The results are shown in Table 3.

### (Comparative Example 27)

According to the same manner as that of Example 10 except that a carrier with no BCG physically adsorbed thereon was used, an amount of induced IFN-γ was measured. The results are shown in Table 3.

### (Comparative Example 28)

According to the same manner as that of Example 11 except that a carrier with no BCG physically adsorbed thereon was used, an amount of induced IFN-γ was measured. The results are shown in Table 3.

### (Comparative Example 29)

According to the same manner as that of Example 12 except that a carrier with no BCG physically adsorbed thereon was used, an amount of induced IFN-γ was measured. The results are shown in Table 3.

### (Comparative Example 30)

According to the same manner as that of Example 9 except that, as a carrier, trade name: Polybeads Polystyrene Microsphere (particle diameter 500 to 600µm, non-porous) manufactured by Polysciences was used, an amount of induced IFN-γ was measured. The results are shown in Table 3.

### (Comparative Example 31)

According to the same manner as that of Example 9 except that, as a carrier, a spherical polystyrene particle (styrene-divinylbenzene copolymer, particle diameter 250 to 750µm, non-porous) manufactured by Moritex Corporation was used, an amount of induced IFN-γ was measured. The results are shown in Table 3.

### (Comparative Example 32)

According to the same manner as that of Example 9 except that, as a carrier, a spherical polymethyl methacrylate particle (particle diameter 750 to 1000µm, non-porous) manufactured by Moritex Corporation was used, an amount of induced IFN-γ was measured. The results are shown in Table 3.

### (Comparative Example 33)

According to the same manner as that of Comparative Example 30 except that a carrier with no BCG physically adsorbed thereon was used, an amount of induced IFN-γ was measured. The results are shown in Table 3.

### (Comparative Example 34)

According to the same manner as that of Comparative Example 31 except that a carrier with no BCG physically adsorbed thereon was used, an amount of induced IFN-γ was measured. The results are shown in Table 3.

### (Comparative Example 35)

According to the same manner as that of Comparative Example 32 except that a carrier with no BCG physically adsorbed thereon was used, an amount of induced IFN-γ was measured. The results are shown in Table 3.

**Table 3**

| | | IFN-γ Concentration (pg/mL) |
|---|---|---|
| Example | 9 | 321 |
| | 10 | 283 |
| | 11 | 262 |
| | 12 | 126 |
| Comparative Example | 25 | 84 |
| | 26 | <10 |
| | 27 | <10 |
| | 28 | <10 |
| | 29 | <10 |
| | 30 | 26 |
| | 31 | 22 |
| | 32 | 19 |
| | 33 | <10 |
| | 34 | <10 |
| | 35 | <10 |

### (Example 13)

A carrier 1 having a bulk volume of 1 mL and BCG (10mg/mL) were kneaded at 4°C for 20 hours in 1mL of a 1 volume % formalin solution (neutral buffered formalin solution, manufactured by Wako Pure Chemical Industries Ltd.)-containing physiological saline, to physically adsorb BCG onto a particle surface. After kneading, this particle was sufficiently washed with a physiological saline, and the particle having a bulk volume of 100µL was filled in a sterilized tube (for 1.5mL, manufactured by Diatron).

According to the same manner as that of Example 1 except that only BCG was not added, and 1.4mL of blood was added, an amount of induced IFN-γ was measured. The results are shown in Table 11.

In addition, immediately after collection of blood of a healthy person used and after culturing of only blood as described above, all values of IFN-γ in plasma were 10pg/mL or lower.

### (Comparative Example 36)

According to the same manner as that of Example 13 except that BCG was not physically adsorbed, an amount of induced IFN-γ was measured. The results are shown in Table 4.

### (Comparative Example 37)

According to the same manner as that of Example 13 except that a carrier 1 was not used, and 1.5mL of blood with BCG added (1mg/mL) was used, an amount of induced IFN-γ was measured.

The results are shown in Table 4.

### (Comparative Example 38)

According to the same manner as that of Example 13 except that, as a carrier, trade name: Polybeads Polystyrene microsphere (particle diameter 500 to 600µm, non-porous) manufactured by Polysciences was used, an amount of induced IFN-γ was measured. The results are shown in Table 4.

### (Comparative Example 39)

According to the same manner as that of Example 13 except that, as a carrier, a spherical polystyrene particle (styrene-divinylbenzene copolymer, particle diameter 250 to 750µm, non-porous) manufactured by Moritex Corporation was used, an amount of induced IFN-γ was measured. The results are shown in Table 4.

### (Comparative Example 40)

According to the same manner as that of Example 13 except that, as a carrier, a spherical polymethyl methacrylate particle (particle diameter 750 to 1000µm, non-porous) manufactured by Moritex Corporation was used, an amount of induced IFN-γ was measured. The results are shown in Table 4.

**Table 4**

| | | IFN-γ Concentration (pg/mL) |
|---|---|---|
| Example | 13 | 256 |
| Comparative Example | 36 | <10 |
| | 37 | 102 |
| | 38 | 16 |
| | 39 | 14 |
| | 40 | 13 |

### (Example 14)

A carrier 1, having a bulk volume of 4mL and a particle surface with BCG physically adsorbed thereon, prepared as in Example 13, was filled in a blood bag (for volume 50mL). 50mL of vein blood containing 15IU/mL of heparin obtained by collection from a healthy person was introduced in this blood bag. This blood bag was incubated at 37°C for 24 hours while mildly stirring. An amount of induced IFN-γ in this blood was measured as in Example 1. The results are shown in Table 5. In addition, immediately after collection of blood of a healthy person used and after culturing of only blood as described above, all values of IFN-γ in plasma were 10pg/mL or lower.

### (Comparative Example 41)

According to the same manner as that of Example 14 except that a carrier 1 with no BCG physically adsorbed thereon was used, an amount of induced IFN-γ was measured. The results are shown in Table 5.

### (Comparative Example 42)

According to the same manner as that of Example 14 except that a carrier 1 was not used and 50mL of blood with 1mg/mL BCG added thereto was used, an amount of induced IFN-γ was measured. The results are shown in Table 5.

### (Comparative Example 43)

According to the same manner as that of Example 14 except that, as a carrier, trade name: Polybeads Polystyrene Microsphere (particle diameter 500 to 600µm, non-porous) manufactured by Polysciences was used, an amount of induced IFN-γ was measured. The results are shown in Table 5.

### (Comparative Example 44)

According to the same manner as that of Example 14 except that, as a carrier, a spherical polystyrene particle (styrene-divinylbenzene copolymer, particle diameter 250 to 750µm, non-porous) manufactured by Moritex Corporation was used, an amount of induced IFN-γ was measured. The results are shown in Table 5.

### (Comparative Example 45)

According to the same manner as that of Example 14 except that, as a carrier, a spherical polymethyl methacrylate particle (particle diameter 750 to 1000µm, non-porous) manufactured by Moritex Corporation was used, an amount of induced IFN-γ was measured. The results are shown in Table 5.

**Table 5**

| | | IFN-γ Concentration (pg/mL) |
|---|---|---|
| Example | 14 | 276 |
| Comparative Example | 41 | <10 |
| | 42 | 81 |
| | 43 | 26 |
| | 44 | 25 |
| | 45 | 22 |

### (Example 15)

BCG was suspended in a physiological saline, and the suspension was diluted with a phosphate buffer, and this was centrifuged at 4000rpm for 15 minutes. The supernatant was discarded, the remaining was suspended in a phosphate buffer again, and the suspension was centrifuged at 4000rpm for 15 minutes. This was repeated three times, then, the material was suspended in a 50% ethanol-containing phosphate buffer, and the suspension was centrifuged at 4000rpm (manufactured by Tomy Seiko Co., Ltd., minor amount high speed centrifuge MRX-150) for 15 minutes. Then, this was suspended in ethanol, and the suspension was centrifuged at 4000rpm for 15 minutes. This was performed two times, and washed with a phosphate buffer again three times. Two mg of this treated BCG was covalently bound with a carrier 1 (bulk volume 1mL) with a carboxyl group introduced therein by a carbodiimide method. The remaining reaction group was reacted with ethanol amine, and this was washed with a phosphate buffer, and then suspended in a phosphate buffer. 100µL of the thus obtained carrier was filled in a sterilized tube.

According to the same manner as that of Example 1 except that only BCG was not added, and an amount of blood to be added was 1. 4mL, an amount of induced IFN-γ was measured. The results are shown in Table 6.

In addition, immediately after collection of blood of a healthy person used and after culturing of only blood as described above, all values of IFN-γ in plasma were 10pg/mL or lower.

### (Comparative Example 46)

According to the same manner as that of Example 15 except that BCG was not covalently bound, an amount of induced IFN-γ was measured. The results are shown in Table 6.

### (Comparative Example 47)

According to the same manner as that of Example 15 except that a carrier 1 was not used, and 1.5mL of blood with 1mg/mL BCG added thereto was used, an amount of induced IFN-γ was measured.

The results are shown in Table 6.

### (Comparative Example 48)

According to the same manner as that of Example 15 except that, as a carrier, trade name: Polybeads Polystyrene Microsphere (particle diameter 500 to 600µm, non-porous) manufactured by Polysciences was used, an amount of induced IFN-γ was measured. The results are shown in Table 6.

**Table 6**

| | | IFN-γ Concentration (pg/mL) |
|---|---|---|
| Example | 15 | 122 |
| Comparative Example | 46 | <10 |
| | 47 | 72 |
| | 48 | 11 |

### (Example 16)

A carrier 1, having a bulk volume of 0.8mL and a particle surface with BCG physically adsorbed thereon, prepared as in Example 13, was filled in a blood bag (for volume 5mL). Five mL of vein blood containing 15IU/mL of heparin obtained by collection of blood from a healthy person was introduced in this blood bag. This blood bag was incubated at 37°C for 24 hours while mildly stirring. An amount of induced TNF-α in this blood was measured according to the same manner as that of Example 1 except that a human TNF-α measuring kit manufactured by ENDOGEN was used. The results are shown in Table 7. In addition, immediately after collection of blood of a healthy person used and after culturing of only blood as described above, all values of TNF-α in plasma were 16pg/mL or lower.

### (Comparative Example 49)

According to the same manner as that of Example 16 except that a carrier 1 with no BCG physically adsorbed thereon was used, an amount of induced TNF-α was measured. The results are shown in Table 7.

**Table 7**

| | TNF-α (pg/mL) |
|---|---|
| Example 16 | 2390 |
| Comparative Example 49 | <156 |

### (Example 17)

A carrier 1, having a bulk volume of 0.8mL and a particle surface with BCG physically adsorbed thereon, prepared as in Example 13, was filled in a blood bag (for volume 5mL). Five mL of vein blood containing 15IU/mL of heparin obtained by collection from a healthy person was introduced in this blood bag. This blood bag was incubated at 37°C for 24 hours while mildly stirring. An amount of induced IL-10 in this blood was measured according to the same manner as that of Example 1 except that a human IL-10 measuring kit manufactured by ENDOGEN was used. The results are shown in Table 8. In addition, immediately after collection of a blood of a healthy person used and after culturing of only blood as described above, all values of IL-10 in plasma were 15pg/mL or lower.

### (Comparative Example 50)

According to the same manner as that of Example 17 except that a carrier 1 with no BCG physically adsorbed thereon was used, an amount of induced IL-10 was measured. The results are shown in Table 8.

**Table 8**

| | IL-10 (pg/mL) |
|---|---|
| Example 17 | 362 |
| Comparative Example 50 | <77 |

### (Example 18)

A carrier 1, having a bulk volume of 0.1mL and a particle surface with BCG physically adsorbed thereon, prepared as in Example 13, was added to a 24-well multiplate (manufactured by Corning). A spleen cell was collected from a C3H/He mouse (8 week old, female, purchased from Nippon SLC) sterilely as much as possible, and adjusted into a RPMI medium (containing 2% bovine fetal serum) to a concentration of 500000 cells/mL. 1.5mL of a suspension of this spleen cell was added to each well of a multiplate, and this was cultured at 37°C under a 5% carbonic acid gas. After 48 hours, the culturing supernatant was recovered from each well, and an IFN-γ concentration in the culture supernatant was measured with a mouse IFN-γ measuring kit (manufactured by ENDOGEN). The results are shown in Table 9.

### (Comparative Example 51)

According to the same manner as that of Example 18 except that a carrier 1 having a particle surface with BCG physically adsorbed thereon, prepared as in Example 13, was not used, a amount of induced IFN-γ was measured. The results are shown in Table 9.

**Table 9**

| | IFN-γ Concentration (ng/mL) |
|---|---|
| Example 18 | 72.1 |
| Comparative Example 51 | <0.15 |

### INDUSTRIAL APPLICABILITY

The cytokine-inducing instrument and the cytokine-inducing method of the present invention can induce a practical level-equivalent amount of cytokine in the presence of a carrier consisting of a water-insoluble porous material using a cytokine-inducing agent, which is epoch making.

Cytokine can be induced at an amount which is fairly higher level than that of a cytokine-inducing agent alone, and the instrument and the method can be suitably used for treating various diseases for which cytokine is effective. Further, in the cytokine-inducing instrument and the cytokine-inducing method of the present invention, cytokine is induced by contact with blood and blood components outside a body and, if necessary, a cytokine-inducing agent which may manifest side effect after cytokine induction is removed and, thereafter, the instrument and the method can be used for treatment.

According to the present invention, therapy which has little side effect and has higher safety than the previous method of administering a cytokine-inducing agent can be attained. For this reason, the cytokine-inducing instrument and the cytokine-inducing method of the present invention can be suitably used for treating a variety of diseases for which induction of cytokine is effective.

## Claims

1. A cytokine-inducing instrument, comprising a cytokine-inducing agent, and a carrier of a water-insoluble porous material.

2. The cytokine-inducing instrument according to claim 1, wherein the porous material has a macro reticular structure.

3. The cytokine-inducing instrument according to claim 1 or 2, wherein the porous material has a pore size distribution of 2 to 2000 Å.

4. The cytokine-inducing instrument according to any one of claims 1 to 3, wherein the porous material consists of a polymer material.

5. The cytokine-inducing instrument according to claim 4, wherein the polymer material consists of at least one kind of polymer material of a polystyrene-based polymer material and an acryl ester-based polymer material.

6. The cytokine-inducing instrument according to any one of claims 1 to 5, wherein the cytokine-inducing agent is bacterium cells and/or a component derived from bacterium cells.

7. The cytokine-inducing instrument according to claim 6, wherein the cytokine-inducing agent is a mycobacteria and/or a component derived from mycobacteria.

8. The cytokine-inducing instrument according to claim 6, wherein the cytokine-inducing agent is hemolytic streptococcus and/or a component derived from hemolytic streptococcus.

9. The cytokine-inducing instrument according to any one of claims 1 to 8, further comprising a container accommodating the cytokine-inducing agent and the porous material.

10. The cytokine-inducing instrument according to any one of claims 1 to 9, which is used for inducing production of cytokine in a cell which can produce cytokine.

11. The cytokine-inducing instrument according to claim 10, wherein the cell which can produce cytokine is a cell derived from a blood or blood component.

12. The cytokine-inducing instrument according to any one of claims 1 to 11, wherein the porous material has cytokine induction enhancing action.

13. A method for induction of cytokine, comprising using a cytokine-inducing instrument as defined in any one of claims 1 to 12.
